(19) Europäisches Patentamt
European Patent Office
Office européen des brevets

(11) Publication number: 0 001 978
A1

(12) # EUROPEAN PATENT APPLICATION

(21) Application number: 78101064.0

(22) Date of filing: 04.10.78

(51) Int. Cl.²: C 07 D 277/06, A 61 K 31/425

(30) Priority: 06.10.77 JP 120794/77

(43) Date of publication of application: 30.05.79
Bulletin 79/11

(84) Designated Contracting States: BE CH DE FR GB NL SE

(71) Applicant: Santen Pharmaceutical Co., Ltd, 163 Shimoshinjo-cho 2 Higashi Yodogawa-ku, Osaka 533 (JP)
(84) Designated Contracting States: BE CH DE FR GB NL SE

(71) Applicant: Yoshitomi Pharmaceutical Industries, Ltd., 35 Hiranomachi 3-chome Higashi-ku, Osaka 541 (JP)
(84) Designated Contracting States: BE CH DE FR GB NL SE

(72) Inventor: Jun-ichi, Iwao, 7-27, Nogami 4, Takarazuka 665 (JP)
Inventor: Masayuki, Oya, 407-668, Oaza Ai, Ibaragi 567 (JP)
Inventor: Toshio, Baba, A-23-105, Tsukumodai 3, Suita 565 (JP)
Inventor: Tadashi, Iso, 2-102, Takabedai 3-2, Tondabayashi 584 (JP)
Inventor: Takehisa, Chiba, 104-1, Uenota Oyamazaki-cho Otokuni-gun, Kyoto 618 (JP)

(74) Representative: von Kreisler, Alek, Dipl.-Chem. et al, Deichmannhaus am Hauptbahnhof, D-5000 Köln 1 (DE)

(54) **A derivative of thiazolidine-4-carboxylic acid, method for the preparation and pharmaceutical compositions comprising the compound.**

(57) The compound, (4R)-3-[(2S)-3-mercapto-2-methylpropanoyl]-4-thiazolidinecarboxylic acid is obtained by treating the corresponding S-benzoyl derivative with acid or alkali. The compound has an inhibitory activity against angiotensin I-converting enzyme and is useful as an antihypertensive agent.

EP 0 001 978 A1

0001978



TITLE MODIFIED
see front page

# Novel Thiazolidine Derivative

This invention relates to (4R)-3-[(2S)-3-mercapto-2-methylpropanoyl]-4-thiazolidinecarboxylic acid represented by the formula (I):

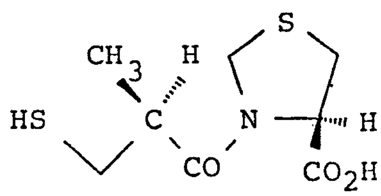

(I)

, method of manufacture thereof, and pharmaceutical composition comprising of the same. The compound (I) of this invention has an inhibitory activity against angiotensin I-converting enzyme and produces a potent antihypertensive action on the renal hypertensive rats. The onset of the compound (1) is rapid after oral administration due to its high lipophilic property. Moreover, the compound has less undesirable adverse effects such as enhancement of carragenin-induced edema and of bradykinin-induced nociception in rats.

The compound of this invention is synthesized by the following method.

S-Benzoyl-3-mercapto-2-methylpropanoyl halide represented by the formula (II); $C_6H_5COSCH_2CH(CH_3)COX$ wherein X is halogen atom, is reacted with (4R)-4-thiazolidinecarboxylic acid by a known method such as Schotten-Baumann reaction to obtain (4R)-3-[(2S)-S-benzoyl-3-mercapto-2-methylpropanoyl]-4-thiazolidinecarboxylic acid. The resulting compound is debenzoylated

with heating , if necessary, under the presence of acid or alkali. As the acid for the reaction, inorganic or organic acid such as hydrochloric acid, sulfuric acid, phosphoric acid and p-toluenesulfonic acid may be used. As the alkali, hydroxide of alkali metal and alkaline earth metal such as sodium hydroxide, potassium hydroxide and calcium hydroxide, or ammonia may be used. As the solvent, the organic solvent or aqueous organic solvent which is not concerned to the reaction may be used. As the solvent which is not concerned to the reaction, lower alcohol such as methanol, ethanol, n-propanol, isopropanol and t-butanol, acetone, tetrahydrofuran, or dioxane may be used. The debenzoylation is well known as ammonolysis in using aqueous ammonia. The reaction is carried out at room temperature, with ice-cooling, or with heating. The objective compound is isolated as follows: the reaction solution containing the objective compound obtained by hydrolysis is concentrated, if necessary, cooled and acidified to crystallize the objective compound. If necessary, the compound is extracted with organic solvent such as ethyl acetate, and the organic layer is dehydrated and concentrated. The obtained compound is purified with the proper solvent such as ethyl acetate and benzene.

## EXAMPLE

(4R)-3-[(2S)-3-Mercapto-2-methylpropanoyl]-4-thiazolidinecarboxylic acid

a) 6.7g of (4R)-4-thiazolidinecarboxylic acid and 12.6g of sodium bicarbonate are dissolved in 150ml of water. To this solution, 12.1g of S-benzoyl-3-mercapto-2-methylpropanoyl chloride is added with stirring under ice-cooling. After the addition, the mixture is stirred for 1 hour under ice-cooling and acidified with dilute hydrochloric acid to obtain oil. The oil is extracted with ethyl acetate, and the ethyl acetate layer is washed with water, dried over anhydrous sodium sulfate and evaporated to dryness. To the oil thus obtained, 100ml of ether is added to obtain crystals. The crystals are filtered to obtain (4R)-3-[(2S)-S-benzoyl-3-mercapto-2-methylpropanoyl]-4-thiazolidinecarboxylic acid, yield 5.0g (29%), mp. 137-139°C (benzene), $[\alpha]_D^{25}$ -172.4° ( c=1, methanol).

Anal. Calcd. for $C_{15}H_{17}NO_4S_2$: C, 53.08; H, 5.05; N, 4.13.

Found: C, 53.21; H, 5.00; N, 4.19.

Dicyclohexylamine salt

mp. 199-200°C (ethyl acetate-ethanol)

$[\alpha]_D^{25}$-109.9° (c=1, methanol)

Anal. Calcd. for $C_{15}H_{17}NO_4S_2 \cdot C_{12}H_{23}N$: C, 62.27; H, 7.74; N, 5.38.

Found: C, 62.32; H, 7.73; N, 5.40.

b) By substituting (2S)-S-benzoyl-3-mercapto-2-methyl-

propanoyl chloride [$[\alpha]_D^{25}$-37.6° (c=1.0, chloroform)], which is prepared from (2S)-S-benzoyl-3-mercapto-2-methylpropanoic acid [mp. 69-71°C, $[\alpha]_D^{25}$-40.4° (c=2.0, methanol)] and thionyl chloride by known method, for S-benzoyl-3-mercapto-2-methylpropanoyl chloride in the procedure of above a), (4R)-3-[(2S)-S-benzoyl-3-mercapto-2-methylpropanoyl]-4-thiazolidinecarboxylic acid is obtained, yield 14.1g (83%), mp. 137-139°C (benzene).

c) To 2.0g of (4R)-3-[(2S)-S-benzoyl-3-mercapto-2-methylpropanoyl]-4-thiazolidinecarboxylic acid, 20ml of conc. ammonia is added and stirred at room temperature for 1 hour. The excess ammonia is removed by concentration in vacuo and by-product, bezamide, is extracted with ethyl acetate. The water layer is acidified with dilute hydrochloric acid and the produced oil is extracted with ethyl acetate. The ethyl acetate layer is washed with water, dried over anhydrous sodium sulfate and evaporated to dryness to obtain (4R)-3-[(2S)-3-mercapto-2-methylpropanoyl]-4-thiazolidinecarboxylic acid, mp. 113-114°C (ether), yield 1.0g (72%), $[\alpha]_D^{25}$-172.0° (c=1, methanol).
Anal. Calcd. for $C_8H_{13}NO_3S_2$: C, 40.83; H, 5.57; N, 5.95.
Found: C, 40.94; H, 5.54; N, 5.94.
Dicyclohexylamine salt
mp. 190-191°C (ethyl acetate)
$[\alpha]_D^{25}$-116.1° (c=1, methanol)

Anal. Calcd. for $C_8H_{13}NO_3S_2 \cdot C_{12}H_{23}N$: C, 57.66; H, 8.71; N, 6.72.

Found: C, 57.63; H, 8.70; N, 6.70.

Reference Example

a) The filtrate of (4R)-3-[(2S)-S-benzoyl-3-mercapto-2-methylpropanoyl]-4-thiazolidinecarboxylic acid in EXAMPLE a) is concentrated to dryness to obtain oil. The oil is applied to silica gel chromatography to obtain (4R)-3-[(2R)-S-benzoyl-3-mercapto-2-methylpropanoyl]-4-thiazolidinecarboxylic acid, yield 4.0g(24%), $[\alpha]_D^{25}$ -19.5° (c=1, methanol).

Dicyclohexylamine salt

mp. 140-141°C (ethyl acetate)

$[\alpha]_D^{25}$ -53.2° (c=1, methanol).

Anal. Calcd. for $C_{15}H_{17}NO_4S_2 \cdot C_{12}H_{23}N$: C, 62.28; H, 7.74; N, 5.38.

Found: C, 62.27; H, 7.70; N, 5.36.

b) To 2.0g of (4R)-3-[(2R)-S-benzoyl-3-mercapto-2-methylpropanoyl]-4-thiazolidinecarboxylic acid, 20ml of conc. ammonia is added and treated in the same manner as mentioned above to obtain (4R)-3-[(2R)-3-mercapto-2-methylpropanoyl]-4-thiazolidinecarboxylic acid, yield 0.8g (58%), $[\alpha]_D^{25}$ -81.5° (c=1, methnol).

Dicyclohexylamine salt

mp. 179-180°C (ethyl acetate-ethanol)

$[\alpha]_D^{25}$ -84.5° (c=1, methanol)

Anal. Calcd. for $C_8H_{13}NO_3S_2 \cdot C_{12}H_{23}N$: C, 57.66; H, 8.71; N, 6.72.

Found: C, 57.60; H, 8.74; N, 6.70.

The potent antihypertensive effect of the compound and its salts of this invention is clear; that is, the inhibitor of angiotensin I-converting enzyme which converts biologically inactive decapeptide, angiotensin I, to active octapeptide, angiotensin II, is found to be useful as an antihypertensive medicine (R.L. Soffer, 1976; M.A. Ondetti, et al., 1977). In view of the above, we investigated the pharmacological activities of the present antihypertensive agent from the aspect of inhibitory activity against the enzyme.

PHARMACOLOGICAL TEST 1

As the method of measurement of angiotensin I-converting enzyme activity, bioassay for the contractile response of isolated smooth muscle or the pressor response of normal animals and biochemical assay for the enzyme isolated from lung or other organs of animals are known. The former is more useful than the latter for the examination of the convertion of angiotensin I to angiotensin II in vitro (R.L. Soffer, 1976.) for the evaluation of the novel compound in the field of medicine.

In the present study, we adopted the bioassay

for contractile response of isolated guinea-pig ileum to angiotensin I.

Measurement of inhibitory activity of angiotensin I-converting enzyme

Preparation of isolated guinea-pig ileum was made and suspended in the organ bath containing 20ml of Tyrode's solution gassed 95% $O_2$ + 5% $CO_2$ at 30°C. The contraction induced by angiotensin I (0.125μg/ml) given at intervals of 10 minutes was recorded on a recticorder (Nihon Koden) for 90 seconds using FD pick up (ST-1T-H, Nihon Koden).

The test compounds were added to the bath 5 minutes before the addition of angiotensin I.

The inhibitory activity of angiotensin I-converting enzyme was calculated by the following formula:

$$\frac{A - B}{A} \times 100$$

A: Contractile intensity of angiotensin I before addition of the compound

B: Contractile intensity of angiotensin I after addition of the compound

The influence of the compounds on the contractile response of isolated guinea-pig ileum to angiotensin II was examined in the same manner as above. Further, augmentation of the contractile response to bradykinin by the test compounds was studied by the above-mentioned method from the fact that kininase II which destroys bradykinin is thought to be identical with angiotensin I-converting enzyme.

The results are shown in Table 1. The test compounds inhibited the contractile response to angiotensin I but not to angiotensin II (inhibition of angiotensin I-converting enzyme), while they enhanced the response to bradykinin (inhibition of kininase II).

PHARMACOLOGICAL TEST 2

The activity of angiotensin I-converting enzyme was measured spectrophotometrically according to the method of D.W. Cushman and H.S. Cheung [Biol. Pharmacol., 20, 1637 (1971)]. That is, the absorbance of hippuric acid was measured, which was liberated by incubating hippuryl-L-histidyl-L-leucine (HHL) as substrate in the presence of angiotensin I-converting enzyme. Angiotensin I-converting enzyme was extracted from rabbit lung by the method of D. Manjusri and R.L. Soffer [J. Biol. Chem., 250, 6762 (1975)].

Measurement of inhibitory activity of angiotensin I-converting enzyme

The reaction mixture is as follows:

100mM phosphate buffer (pH 8.3)
300mM sodium chloride
5mM HHL
$10^{-4}$ - $10^{-8}$M enzyme inhibitor
0-10mU enzyme

0.25ml of the above mixture was incubated at 37°C for 30 minutes and the reaction was stopped by adding 0.25ml of 1 N hydrochloric acid. To this solution, 1.5ml of ethyl acetate was added in order to extract hippuric acid. 1.0ml of ethyl acetate layer was collected and evaporated to dryness and the residue obtained was dissolved in 1.0ml of water and the absorbance thereof was measured at 228nm.

The inhibitory activity of angiotensin I-converting enzyme was calculated by the following formula:

$$\text{Inhibitory percentage} = \frac{A-B}{A} \times 100$$

A: Absorbance of the reacrion solution without the compound

B: Absorbance of the reaction solution in the presence of the compound

Molar concentration of the compound producing 50% inhibition of angiotensin I-converting enzyme ($IC_{50}$)

The inhibitory activity of the compound was measured

at the concentration of 1 x $10^{-4}$M to 1 x $10^{-8}$M and $IC_{50}$ at each concentration was calculated.

The results are shown in Table 2.

Test compounds are as follows:

Compound A: (4R)-3-[(2S)-3-mercapto-2-methylpropanoyl]-4-thiazolidinecarboxylic acid (the compound of this invention)

Compound B: (4R)-3-[(2R)-3-mercapto-2-methylpropanoyl]-4-thiazolidinecarboxylic acid

Table 1. Inhibitory activity of the compounds against angiotensin I-converting enzyme

| Compound | Final concentration (M) | Inhibition to A-I (%) | Augmentation to BK (%) | Inhibition to A-II (%) |
|---|---|---|---|---|
| A | $10^{-5}$ | - | - | 0 |
| | $10^{-6}$ | 72.6 | - | - |
| | $10^{-7}$ | 38.1 | 125.3 | - |
| | $10^{-8}$ | - | 60.4 | - |
| | $10^{-9}$ | - | 22.1 | - |
| B | $10^{-5}$ | 59.3 | - | 0 |
| | $10^{-6}$ | 24.7 | - | - |
| | $10^{-7}$ | - | 78.3 | - |
| | $10^{-8}$ | - | 16.8 | - |

A-I : angiotensin I
A-II: angiotensin II
BK : bradykinin

Table 2. Inhibitory activity of the compounds against angiotensin I-converting enzyme

| | Inhibitory percentage | | | | | | | | $IC_{50}$* | |
|---|---|---|---|---|---|---|---|---|---|---|
| Concentration M | $10^{-8}$ | $10^{-7}$ | $5\times10^{-7}$ | $10^{-6}$ | $5\times10^{-6}$ | $10^{-5}$ | $5\times10^{-5}$ | $10^{-4}$ | M | μg/ml |
| Compound A | 0 | 21 | 84 | 100 | - | - | - | - | $3.20\times10^{-7}$ | 0.075 |
| Compound B | - | 8 | - | 43 | 81 | 98 | - | - | $1.32\times10^{-6}$ | 0.310 |

* Concentration of the compound producing 50% inhibition of the enzyme activity.

Toxicity test

Acute toxicity of the compound of this invention, (4R)-3-[(2S)-3-mercapto-2-methylpropanoyl]-4-thiazolidinecarboxylic acid, is shown in Table 3. As seen in the table, the compound of this invention shows lower toxicity.

(Experimental animals)

The male ddy-SLC strain rats (5 weeks of age, weighing 20-25g) were placed in a breeding room of constant temperature and humidity (24±1°C, 55±5%) and fed freely pellet diet (CE-2, Clea Japan Inc.) and water for a week. The rats showing the normal growth were selected for the experiment.

(Method of administration)

30% suspension or 30% solution (pH 7.0) was administered orally, subcutaneously or intravenously and the toxicity was calculated by the Miller and Tainter method.

Table 3.

$LD_{50}$ (mg/kg)

| Route | Rats | Mice |
|---|---|---|
| p.o.* | 6600±975 | 8800±716 ( > 10000**) |
| s.c.** | 3650±244 | 2950±183 |
| i.v.** | 3260±234 | 2300±174 |

* 30% suspension

** 30% solution (pH 7.0)

It is found from the above pharmacological tests that the compound (I) of this invention is useful as an antihypertensive agent. The compound can be given with the combination of diuretics as other antihypertensive agents. The compound can be administered either orally or parenterally. The dosage forms are tablet, capsule, granule, powder, suppository, injection, etc. In the treatment of hypertension, these preparations can contain not only general fillers but also antihypertensive agents such as reserpine, α-methyldopa, guanethidine, clonidine, hydralazine, etc. The dosage varies according to symptoms and administration forms, etc. But, usual dosage range is 1 to 5000mg a day, preferably 10 to 1000mg once a day or divided into a few doses.

The follwings are examples of formulation.

1. Oral drug

a. tablet

| | |
|---|---|
| compound of formula (I) | 30mg |
| lactose | 150mg |
| crystalline cellulose | 50mg |
| calcium carboxymethylcellulose | 7mg |
| magnesium stearate | 3mg |
| Total | 240mg |

The tablets may be treated with the common film-coating and further with sugar-coating.

b. granule

| | |
|---|---|
| compound of formula (I) | 30mg |
| polyvinylpyrrolidone | 25mg |
| lactose | 385mg |
| hydroxypropylcellulose | 50mg |
| talc | 10mg |
| Total | 500mg |

c. powder

| | |
|---|---|
| compound of formula (I) | 300mg |
| lactose | 230mg |
| starch | 440mg |
| colloidal silica | 30mg |
| Total | 1000mg |

d. capsule

| | |
|---|---|
| compound of formula (I) | 30mg |
| lactose | 102mg |
| crystalline cellulose | 56mg |
| colloidal silica | 2mg |
| Total | 190mg |

2. Injection

1 to 30mg of compound of formula (I) is contained in 1 ml of the aqueous solution (pH 6.5-7.0).

What we claim is:

1) (4R)-3-[(2S)-3-Mercapto-2-methylpropanoyl]-4-thiazolidinecarboxylic acid.

2) Method of preparation of (4R)-3-[(2S)-3-mercapto-2-methylpropanoyl]-4-thiazolidinecarboxylic acid, which comprises treating (4R)-3-[(2S)-S-benzoyl-3-mercapto-2-methylpropanoyl]-4-thiazolidinecarboxylic acid with acid or alkali.

3) A pharmaceutical composition comprising the compound of Claim 1, in combination with a pharmaceutically acceptable inert carrier, said compound being present in an effective amount for the treatment of hypertensive diseases.

European Patent Office

# EUROPEAN SEARCH REPORT

0001978

Application number

EP 78 101 064.0

## DOCUMENTS CONSIDERED TO BE RELEVANT

| Category | Citation of document with indication, where appropriate, of relevant passages | Relevant to claim |
|---|---|---|
| PX | DE - A - 2 752 719 (SQUIBB) * claims 1, 17 , 18; page 10, lines 18 bis 23; example 5 * | 1-3 |
| | -- | |
| - | FR - A - 1 559 851 (ETUDES ET RECHERCHES MEDICALES APPLIQUEES) * examples * | 2 |
| | -- | |
| A | DE - A - 2 116 629 (INSTITUTUL DE BIOCHEMIE) | |
| | -- | |
| A | DE - A - 2 354 143 (DUFOUR, SOCIETE D'ETUDES) & GB - A - 1 410 138 | |
| | -- | |
| A | GB - A - 1 265 840 (ACADEMIA REPUBLICII SOCIALISTE ROMANIA) | |
| | ---- | |

**CLASSIFICATION OF THE APPLICATION (Int. Cl.²)**

C 07 D 277/06
A 61 K 31/425

**TECHNICAL FIELDS SEARCHED (Int.Cl.²)**

A 61 K 31/425
C 07 D 277/06

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 29-12-1978 | FROELICH |